(19) Europäisches Patentamt
European Patent Office
Office européen des brevets

(11) Veröffentlichungsnummer: **0 262 583**
**A1**

(12) # EUROPÄISCHE PATENTANMELDUNG

(21) Anmeldenummer: **87113940.8**

(51) Int. Cl.[4]: **A61K 9/22** , A61K 37/24

(22) Anmeldetag: **24.09.87**

Die Bezeichnung der Erfindung wurde geändert (Richtlinien für die Prüfung im EPA, A-III, 7.3).

(30) Priorität: **13.12.86 DE 3642662**
**02.10.86 DE 3633551**

(43) Veröffentlichungstag der Anmeldung:
**06.04.88 Patentblatt 88/14**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Humke, Rainer, Dr.**
**Amselweg 5**
**D-6233 Kelkheim (Taunus)(DE)**
Erfinder: **Seidel, Heinz-Rüdiger**
**Im Kirschenfeld 15**
**D-6370 Oberursel/Taunus(DE)**
Erfinder: **von Rechenberg, Wolfrad, Dr.**
**Kirberger Strasse 39**
**D-6251 Kaltenholzhausen(DE)**

(54) **Calciumacetat-Glycerin-Addukt enthaltende parenterale Depotzubereitungen von regulatorischen Peptiden.**

(57) Es werden regulatorische Peptide enthaltende, parenterale Depotzubereitungen auf Basis von Poly-D-(-)-3-hydroxybuttersäure, die Calciumacetat-Glycerin-Addukt, eine Molekülverbindung aus 1 Mol Calciumacetat und 2 Mol Glycerin enthalten sowie ein Verfahren zu ihrer Herstellung beschrieben. Das Addukt wird als die Wirkstoff-Freigabe beeinflussender Hilfsstoff verwendet

EP 0 262 583 A1

# Parenterale Depotzubereitungen von regulatorischen Peptiden enthaltend Calciumacetat-Glycerin-Addukt, Verfahren zu ihrer Herstellung sowie die Verwendung des Addukts

Die Erfindung betrifft parenterale Depotzubereitung, die regulatorische Peptide als Wirkstoff. Poly-D-(-)-3-hydroxybuttersäure als biologisch abbaubaren Trägerstoff und Calciumacetat-Glycerin-Addukt als die Wirkstoff-Freigabe beeinflussenden Hilfsstoff enthalten, Verfahren zur Herstellung dieser Zubereitungen sowie die Verwendung des Addukts zur Beeinflussung der wirkstoff-Freigabe aus parenteralen Depotzubereitungen enthaltend regulatorische Peptide und Poly-D-(-)-3-hydroxybuttersäure.

Die therapeutische Anwendung von Arzneizubereitungen mit kontrollierter und verzögerter Freisetzung hat sich gegenüber solchen mit nicht verzögerter Wirkstoff-Freigabe vielfach als vorteilhaft herausgestellt, führt sie doch zu länger anhaltenden und gleichmäßigeren statt schwankenden Blutspiegeln und verringert die Applikationshäufigkeit. Insbesondere bei parenteraler Applikation vereinfachen Depotarzneiformen die Therapie oder gestalten sie erst sinnvoll, wie z.B. bei der Anwendung von Wirkstoffen mit Peptidstruktur.

Um die Freigabe eines Wirkstoffs aus einer Arzneiform zu steuern, kann man ihn z.B. entweder mit einer Polymermembran umhüllen oder ihn in ein Polymer einbetten. Dabei ist es für eine parenterale Anwendung günstig, biologisch abbaubare Polymere zu verwenden, die ein späteres Entfernen der Depotzubereitung nach erfolgter Freisetzung des Wirkstoffs unnötig machen.

Derartige parenterale Depotzubereitungen mit konstanter Wirkstoff-Freisetzung auf Basis biologisch abbaubarer Polymere ist schon beschrieben worden. Beispielsweise sind in der Europäischen Patentanmeldung mit der Veröffentlichungs-Nr. 0133988 implantierbare Zubereitungen genannt, die regulatorische Peptide, wie das LH-RH-Analogon Buserelinacetat, in einer biologisch abbaubaren aus Poly-3-hydroxybuttersäure bestehenden Matrix enthalten. Aus diesen Implantaten wird das Peptid über viele Wochen konstant freigegeben.

Die in der genannten Anmeldung aufgeführten Implantat-Zubereitungen werden dabei durch direkte und einzelne Verpressung erhalten. Das Verfahren ist folglich umständlich und zeitraubend.

Eine wesentliche Erleichterung der Herstellung besteht darin, Peptid und Poly-3-hydroxybuttersäure zu mischen und die Mischungen zu granulieren. Das dabei gebildete frei fließende Granulat kann bequem kontinuierlich verpreßt werden. Es wurde jedoch festgestellt, daß die durch Mischung, Granulation und Verpressung hergestellten Zubereitungen keine gleichmäßige Freigabe aufwiesen.

Es wurde nun gefunden, daß bei den parenteralen peptidhaltigen durch Mischung, Granulation und Verpressen hergestellten Depotzubereitungen eine gleichmäßige oder nahezu gleichmäßige Freigabe dadurch erreicht werden kann, daß man einen weiteren Hilfsstoff, nämlich Calciumacetat-Glycerin-Addukt, zusetzt.

Die beiden Komponenten des Adduktes sind physiologisch verträglich. Das Calcium-Glycerin-Addukt ist eine Molekülverbindung zwischen 1 Mol Calciumacetat und 2 Mol Glycerin der Formel I

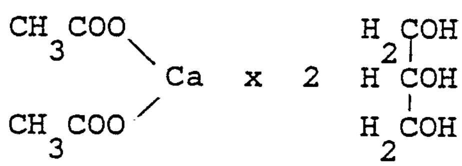

I

(vgl. z.B. Chemical Abstracts Vol. 52, 3582 a (1958))

Entsprechend den Angaben in der Literatur erhält man das Addukt indem man eine Mischung aus 2 Mol Glycerin und einem $C_1$-$C_3$-Alkohol, vorzugsweise Methanol, in der Wärme mit 1 Mol Calciumacetat versetzt, die Mischung bis zur Siedetemperatur erhitzt, danach abkühlt und das Addukt abtrennt.

Das Calciumacetat-Glycerin-Addukt beeinflußt die Wirkstoff-Freigabe aus parenteralen Depotzubereitungen. Die Erfindung betrifft daher parenterale Depotzubereitungen, die regulatorische Peptide als Wirkstoff, Poly-D-(-)-3-hydroxybuttersäure als biologisch abbaubaren Trägerstoff und Calciumacetat-Glycerin-Addukt als die Wirkstoff-Freigabe beeinflussenden Hilfsstoff enthalten. Die Erfindung betrifft ferner auch die Verwendung des Addukts zur Beeinflussung der Wirkstoff-Freigabe aus den genannten Zubereitungen.

Unter parenteralen Depotzubereitungen werden in den vorstehenden und folgenden Ausführungen implantierbare oder injizierbare feste Zubereitungen unterschiedlicher Form verstanden.

Regulatorische Peptide, auch als Peptidhormone bezeichnet, sind physiologisch wirksame endogene Peptide. Sie sind z.B. in Wasser und niedermolekularen Alkoholen gut löslich. Als Vertreter, die die erfindungsgemäßen Depotzubereitungen enthalten können, seien genannt Oxytocin, Vasopressin, Adrenocorticotropes Hormon, Prolactin, Luteinisierendes Hormon-Releasing Hormon (LH-RH), Insulin, Glucagon, Gastrin, Sekretin und Somatostatin. Als regulatorische Peptide werden auch ihre Analoga und ihre physiologisch verträglichen Salze verstanden. Vorzugsweise enthalten die Zubereitungen Buserelin oder Buserelin Acetat.

Poly-D-(-)-3-hydroxybuttersäure ist ein mikrobiologisch gewonnenes Polymer (vgl. z.B. J.N. Baptist et al., SPE Transactions, Oktober 1964, Seiten 245-250). Sein Molekulargewicht liegt im Bereich von 25 000 bis 1 000 000.

Der Peptidgehalt in den erfindungsgemäßen Zubereitungen liegt, bezogen auf ihr Gesamtgewicht, bei 0,05 - 15 %, vorzugsweise 0,1 - 10%.

Der Anteil an Addukt in der erfindungsgemäßen Zubereitungen beträgt, bezogen auf ihr Gesamtgewicht, 1 - 30 %, vorzugsweise 2 - 10 %.

Der Zusatz von Calciumacetat-Glycerin-Addukt bei der Herstellung der Depotzubereitungen führt zur gewünschten gleichmäßigen Freigabe des Wirkstoffs. Es wird verhindert, daß die Depotzubereitung vorzeitig erschöpft ist und bei langdauernder Behandlung in kurzen Zeitabstanden ersetzt werden muß.

Die Beeinflussung der Wirkstoff-Freisetzung durch das Calciumacetat-Glycerin-Addukt erfolgt durch Erhöhung der Porosität der Depotzubereitung, da es als gut wasserlösliche Substanz durch Herauslösen freigesetzt wird. Dabei konkurriert es im Falle hoher Anteile an Peptid in der Depotzubereitung mit diesen, so daß die initiale Freisetzung des Peptids verringert wird und das Freigabeprofil in Richtung eines gleichmäßigeren Verlaufs verschoben wird.

Bei geringen Wirkstoffmengen bewirkt ein Zusatz von Calciumacetat-Glycerin-Addukt überhaupt erst eine ausreichende Porosität, was zur Folge hat, daß eine genügende, d.h. sich in vivo auswirkende Wirkstoff-Freisetzung, stattfindet. Je nach Anteil an Peptid in der Depotzubereitung erhält man Freisetzungszeiten von wenigen Tagen bis zu mehreren Wochen.

Durch Wahl geeigneter Anteile an regulatorischem Peptid und Calciumacetat-Glycerin-Addukt in der Depotzubereitung ist es möglich, Freisetzungsrate und Freisetzungsdauer zu steuern. Die Verwendung von Calciumacetat-Glycerin-Addukt in parenteralen Depotzubereitungen stellt daher eine wertvolle Methode zur Kontrolle der Wirkstoff-Freisetzung dar.

Die Erfindung betrifft weiterhin Verfahren zur Herstellung von parenteralen Depotzubereitungen zur kontrollierten Freisetzung von regulatorischen Peptiden.

Das Verfahren zur Herstellung derartiger Depotzubereitungen ist dadurch gekennzeichnet, daß man die Peptidwirkstoffe, Calciumacetat-Glycerin-Addukt und Poly-3-hydroxybuttersäure innig vermischt, die Mischung nach Granulation mit einem geeigneten organischen Lösungsmittel, wie z.B. Methylenchlorid oder mit Hilfe einer Lösung der Poly-3-hydroxybuttersäure in dem organischen Lösungsmittel, zu Formkörpern verpreßt.

Die Preßlinge können ganz unterschiedliche Formen, wie Zylinder. Stäbchen und Plättchen besitzen und implantiert oder injiziert (z.B. mit Hilfe eines Trochars) werden. Im Gegensatz zu den Implantaten entsprechend EP-A-0133988 kann die Verpressung zu Formkörpern auf modernen Maschinen kontinuierlich erfolgen, da das Granulat gute Fließeigenschaften hat. Damit ist das Verfahren wesentlich wirtschaftlicher als das beschriebene.

Die folgenden Beispiele erläutern die Erfindung

Beispiel 1

Herstellung von Implantat-Tabletten

Durch Mischen, Granulation mit Methylenchlorid und Verpressung wurden Implantat-Tabletten hergestellt, bestehend aus 5 mg Buserelin-Acetat, 5 mg Calciumacetat-Glycerin-Addukt und 40 mg Poly-3-hydroxybuttersäure.

Die Wirkstoff-Freigabe wurde in Puffer pH 7,4 im Vergleich zu einem adduktfreien Implantat bestimmt, wobei das Medium (2 ml)täglich gewechselt wurde. Die Freigaberaten wurden nur an Tag 1, 7, 14 usw. bestimmt. Sie betrugen:

| | Implantat mit Addukt | Implantat ohne Addukt |
|---|---|---|
| Tag 1 | 576 µg/Tag | 1466 µg/Tag |
| Tag 7 | 192 µg/Tag | 189 µg/Tag |
| Tag 14 | 151 µg/Tag | 86 µg/Tag |
| Tag 21 | 98 µg/Tag | 12 µg/Tag |
| Tag 28 | 49 µg/Tag | - |

Die addukthaltige Zubereitung erlaubt, am Tier einen wirksamen Buserelin-Spiegel über 4 Wochen aufrecht zu erhalten.

Beispiel 2

Herstellung von Implantat-Tabletten

Es wurden, wie in Beispiel 1 angegeben, 50 mg schwere Implantat-Tabletten aus Poly-3-hydroxybuttersäure hergestellt, enthaltend 50 µg Gonadorelin und 2,5 mg Calciumacetat-Glycerin-Addukt.

Die Wirkstoff-Freigabe wurde in Puffer pH 7,4 bestimmt. Als Vergleich diente ein adduktfreies Implantat (Anordnung wie in Beispiel 1 angegeben). Die Freigaberaten betrugen:

| | Implantat mit Addukt | Implantat ohne Addukt |
|---|---|---|
| Tag 1 | 4,72 µg | 43,3 ng |
| Tag 2 | 1,97 µg | 11,6 ng |
| Tag 3 | 2,43 µg | 7,4 ng |
| Tag 4 | 2,29 µg | <1 ng |
| Tag 5 | 1,38 µg | |

Mit den Addukt enthaltenden Implantaten gelingt es, bei azyklischen Schafen Ovulationen auszulösen.

## Ansprüche

1. Parenterale Depotzubereitungen, die regulatorische Peptide als Wirkstoff und Poly-D-(-)-3-hydroxybuttersäure als biologisch abbaubaren Trägerstoff enthalten, dadurch gekennzeichnet, daß sie Calciumacetat-Glycerin-Addukt der Formel I

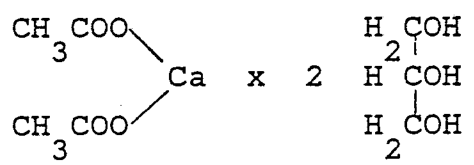

I

als die Wirkstoff-Freigabe beeinflussenden Hilfsstoff enthalten.

2. Parenterale Depotzubereitung gemäß Anspruch 1, dadurch gekennzeichnet, daß sie Buserelin oder Buserelin-Acetat als Wirkstoff enthält.

3. Verfahren zur Herstellung einer parenteralen Depotzubereitung, dadurch gekennzeichnet, daß man ein regulatorisches Peptid, Calciumacetat-Glycerin-Addukt und Poly-D-(-)-3-hydroxybuttersäure innig vermischt und die Mischung nach Granulation mit einem geeigneten Lösungsmittel oder mit Hilfe einer Lösung der Poly-D-(-)-3-hydroxybuttersäure in dem organischen Lösungsmittel zu Formkörpern verpreßt.

4. Verwendung des Calciumacetat-Glycerin-Addukts zur Beeinflussung der Wirkstoff-Freigabe aus parenteralen Depotzubereitungen enthaltend regulatorische Peptide als Wirkstoff und Poly-D-(-)-3-hydroxybuttersäure als biologisch abbaubaren Trägerstoff.

Europäisches Patentamt

# EUROPÄISCHER RECHERCHENBERICHT

Nummer der Anmeldung

EP 87 11 3940

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 92, Nr. 12, 24. März 1980, Seite 70, Zusammenfassung Nr. 95642x, Columbus, Ohio, US; J. BULACOVSCHI et al.: "Studies on the manufacture and characterization of films from collagen solutions. Part I.", & BUL. INST. POLITEH. IASI, SECT. 7 1978, 24(1-4), 17-23 | 1-4 | A 61 K 9/22<br>A 61 K 37/24 |
| A | CHEMICAL ABSTRACTS, Band 103, Nr. 22, 2. Dezember 1985, Seite 100, Zusammenfassung Nr. 179975s, Columbus, Ohio, US; M.S. NIEUWENHUIZEN et al.: "Synthesis and calcium complexation of polycarboxylic acids. Synthesis and calcium complexation of a series of low-molecular weight polycarboxylic acids: derivatives of oxydiacetate and ethylene glycol diacetate", & TENSIDE DETERG. 1985, 22(5), 247-51 | 1-4 | |

| RECHERCHIERTE SACHGEBIETE (Int. Cl.4) |
|---|
| A 61 K |

Der vorliegende Recherchenbericht wurde für alle Patentansprüche erstellt

| Recherchenort | Abschlußdatum der Recherche | Prüfer |
|---|---|---|
| DEN HAAG | 08-01-1988 | FOERSTER W.K. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0401)